# EUROPEAN PATENT APPLICATION

(11) **EP 1 811 299 A1**
(43) Date of publication of application: **25.07.2007**
(21) Application number: 05799283.6
(22) Date of filing: 25.10.2005
(51) Int. Cl.: G01N 33/53, C12M 1/00, G01N 37/00, C12N 15/09

(54) **BIOASSAY EQUIPMENT AND BIOASSAY METHOD**

(30) Priority: 04.11.2004 JP 2004320695
(71) Applicant: SONY CORPORATION, Minato-ku Tokyo 108-0075 (JP)
(72) Inventor: ITO, Tatsumi, c/o Sony Corporation, Tokyo 141 (JP); TAKEDA, Minoru, c/o Sony Corporation, Tokyo 141 (JP); FURUKI, Motohiro, c/o Sony Corporation, Tokyo 141 (JP); YAMANE, Ryoichi, c/o Sony Corporation, Tokyo 141 (JP); NAKAJIMA, Toshihiro, c/o Sony Corporation, Tokyo 141 (JP)
(74) Representative: Robinson, Nigel Alexander Julian
(86) International application number: PCT/JP2005/019589
(87) International publication number: WO 2006/049041

(57) **Abstract**

An object is to prevent changes through drying in the concentrations of a medium stored or held in reaction regions that provide fields for an interaction between substances, and also, precipitation or sticking of the substances in the medium. Provided is a bioassay system 2, which is equipped at least with medium feeding means for feeding, to reaction regions R that provide fields for an interaction such as hybridization between substances, a medium that contains one of the substances to be involved in the interaction and water replenishing means for automatically replenishing water into the reaction regions as needed. The system 2 may be in such a construction as having volume detecting means capable of automatically detecting a volume of the medium held in each of the reaction regions R.

## Description

### [Technical Field]

This invention relates to a bioassay system and bioassay method. More specifically, the present invention is concerned with a bioassay system and bioassay method contrived to prevent changes through drying in the concentrations of a medium stored or held in reaction regions that provide fields for an interaction between substances.

### [Background Art]

In recent years, integrated bioassay plates with desired DNAs microarrayed thereon by microarray technologies and generally called "DNA chips" or "DNA microarrays" (hereinafter collectively called "DNA chips") have found increasing utility in gene mutation analyses, SNPs (single-base polymorphisms), gene expression frequency analyses, and the like, and have begun to find broad applications in drug developments, clinical diagnoses, pharmacogenomics, evolution research, forensic medicine, and other fields. A "DNA chip" is characterized by the feasibility of a comprehensive analysis of hybridization, because a wide variety of numerous DNA oligonucleotides chains or cDNAs (complementary DNAs) are integrated on a glass substrate or silicon substrate.

In addition to the above-described DNA chips, diverse sensor chips have been developed including protein chips useful in detecting protein-associated interactions. In general, this sensor chip is the technology that reaction regions, which meet such conditions as providing fields for an interaction (for example, hybridization) between substances such as biomolecules, are arranged beforehand on a substrate and the existence or non-existence or the extent of an interaction between a probe substance immobilized beforehand in each of the reaction regions and a target substance is detected by using a measurement theory such as the fluorescence signal detection, the surface plasma resonance theory, or the quartz crystal theory.

According to this sensor chip technology, extremely small amounts of a medium (for example, solution) are handled. Drying of the medium during an assay of an interaction, therefore, causes a change in the concentration of a substance in the medium and its precipitation, thereby adversely affecting the accuracy of the measurement. Upon conducting an assay for the analysis of an interaction, it is thus necessary to take such a countermeasure as setting an environment of suitable humidity and temperature or enclosing each reaction region to prevent the evaporation of water.

For example, Japanese Patent Laid-open No. 2002-36302 discloses the technology that the humidity is set at such a level as making a sample solution hardly evaporate with steam from a steam generator arranged as an accessory to a microarray system in order to resolve the problem that the quality of a microarray does not remain stable if the sample solution evaporates during sample spotting work upon preparation of the microarray (upon immobilization of probes).

Further, Japanese Patent Laid-open No. 2003-079377 discloses the technology that a predetermined amount or greater of a polyhydric alcohol is included beforehand in a gel to prevent evaporation of water from the gel or separation of the gel from a substrate.

For the reduction of the evaporation (rate) of water from a reaction region, only two approaches are theoretically conceivable, one being (1) to raise the relative humidity and the other (2) to lower the temperature so that the saturated vapor pressure is lowered.

It may, therefore, be contemplated to adopt such an approach as maintaining under a high-humidity environment the entire atmosphere upon allowing an assay step to proceed in a reaction region. There are, however, limitations to its effects. This approach also develops problems such as an increase in machine and equipment cost for the maintenance of a high humidity and the indispensability of maintenance measures.

It may be effective to enclose each reaction region, in which a sample solution is held, by a cover member or the like. It, however, takes time until the feeding (for example, dropping) work of a probe substance or target substance into the respective reaction regions, which are arranged in a large number on a substrate, is all completed. Accordingly, the concentration of a substance in each reaction zone changes moment after moment with time until its cover is applied.

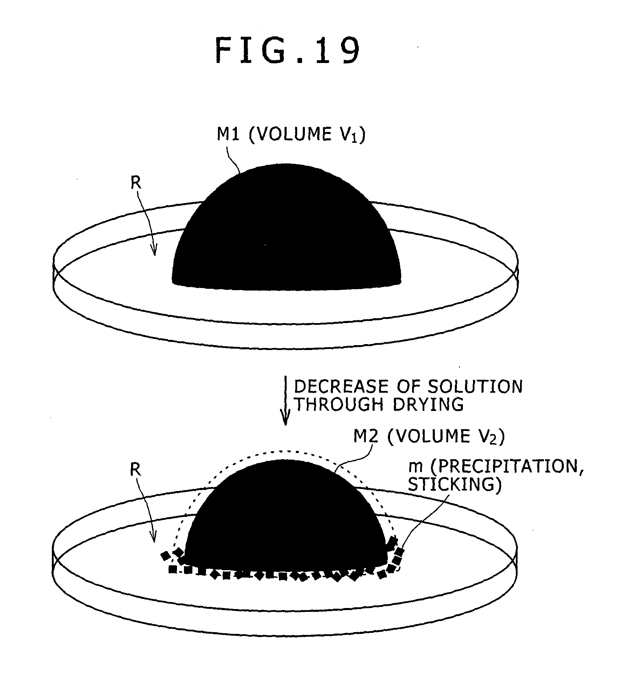

As illustrated by way of example in FIG. 19, a medium M1 fed in a predetermined amount into a given reaction region R dries with time, and its initial volume V1 gradually decreases to give a medium M2 of a volume V2 (V2 < V1). Due to this volume change of the medium, a problem arises in that the concentration of a substance m (probe substance, target substance, or the like) in the reaction region R changes or the substance m precipitates or sticks in the reaction region R. As a result, variations occur in the concentration of the substance among the reaction regions R, thereby adversely affecting the detection accuracy.

The present invention, therefore, has as its principal objects the provision of a bioassay system and bioassay method, which can replenish water for that lost from a medium in each of reaction regions, which provide fields for an interaction between substances, to prevent a change in the concentration of a substance contained in each reaction region or the precipitation of sticking of the substance in the medium, which would otherwise take place as a result of drying of the medium stored or held in each reaction region.

### [Disclosure of Invention]

In the present invention, the following "bioassay system" and "bioassay method" are provided.

The "bioassay system" according to the present invention includes at least: medium feeding means for feeding, to reaction regions that provide fields for an interaction such as hybridization between substances, a medium that contains one of the substances to be involved in the interaction, and water replenishing means for automatically replenishing water into the reaction regions as needed.

According to this system, it becomes possible, for example, to replenish water to each reaction region at a desired timing in a volume equal to that lost from the medium fed into the reaction region. This replenishment makes it possible to maintain the concentration of the substance at a desired concentration in the medium, to make even the concentration of the substance in the medium among numerous reaction regions, and to effectively prevent the precipitation or sticking of the substance, which would otherwise take place by overdrying.

The bioassay system may also be contrived to further include volume detecting means capable of automatically detecting a volume of the medium held in each of the reaction regions such that based on information on a volume change available from the volume detecting means, water can be replenished in an amount corresponding to that lost through drying to the each reaction region via the water replenishing means. Although not limited in particular, the volume detecting means can suitably adopt, for example, means that extracts a profile of the medium held in the each reaction region from a camera output image and calculates the volume of the medium from dimensions of a meniscus profile of the medium.

Next, the bioassay method according to the present invention includes performing a step of feeding, to reaction regions that provide fields for an interaction between substances, a medium, the medium containing one of the substances to be involved in the interaction, and water via different routes by one of the following procedures (1) and (2): (1) feeding the medium that contains the one of the substances to be involved in the interaction, and then replenishing the water, and (2) replenishing the water, and then feeding the medium that contains the one of the two substances to be involved in the interaction.

This method makes it possible to replenish water for that lost through drying after a medium with a substance such as a probe substance or target substance contained therein has been fed (the procedure (1)), or to feed a medium with a substance such as a probe substance or target substance contained therein is fed after water has been replenished (the procedure (2)). The procedure (2) is particularly effective for preventing the deposition or sticking of the substance at edges or boundary areas.

Definitions for certain principal technical terms employed in the present invention will now be described.

The term "interaction" broadly means chemical boding or dissociation including non-covalent bonding, covalent bonding, and hydrogen bonding between substances, and broadly includes, for example, hybridization as complementary bonding between nucleic acids (nucleotide chains) and bonding, association, or the like between high molecular substances, between a high molecular substance and a low molecular substance, or between low molecular substances. "Hybridization" means a reaction that forms a complementary chain (double-stranded chain) between nucleotide chains having complementary base sequence structures.

The term "reaction region" means an area or space that can provide a reaction field for hybridization or another interaction. Illustrative can be a reaction field that has the shape of a reaction well capable of storing a liquid phase, gel, or the like. It is to be noted that an interaction to be effected in such a reaction region shall not be narrowly limited insofar as the interaction is in conformity with the object and effects of the present invention.

The term "medium" means a water-containing medium which contains substances such as a substance (probe substance or target substance) to be involved in an interaction and a substance, such as an intercalator, to be used for the detection of the interaction.

The term "nucleic acid" means a polymer (nucleotide chain) of the phosphate ester of a nucleoside with a purine or pyrimidine base and a sugar bonded together via a glycoside linkage, and broadly encompasses oligonucleotides including probe DNAs, polynucleotides, DNAs (full lengths or their fragments) formed by polymerization of purine nucleotides and pyrimidine nucleotides, cDNAs (complementary probe DNAs) obtained by reverse transcription, RNAs, polyamide nucleotide derivatives (PNAs), and the like.

According to the present invention, the concentration of a substance can be adjusted within each of reaction regions arranged on a plate provided for a bioassay. It is, therefore, possible to perform a high-accuracy bioassay process. Further, it becomes no longer necessary to maintain high the internal humidity of a constant-humidity chamber, thereby making it possible to realize a system of reduced size and cost. By using means that automatically detects the amount of a medium such as a solution in each reaction region, the adjustment of the concentration of the substance can be facilitated.

### [Brief Description of Drawings]

[FIG. 1] FIG. 1 is a cross-sectional view showing the construction of members in a plate with reaction regions to which the bioassay system and method according to the present invention can be applied.
[FIG. 2] FIG. 2 is a perspective view illustrating how an upper plate (hereinafter called "the cover") 12 is put on a lower plate 11.
[FIG. 3] FIG. 3 is a cross-sectional view depicting the layered construction of a plate 1 obtained by the superimposition of the plate 11 and the cover 12.
[FIG. 4] FIG. 4 is a diagram showing a state that the lower plate 11 is processed at the position of a feed module 2a in the system 2.
[FIG. 5] FIG. 5 is a diagram illustrating a state that the plate 1 is processed at the position of a reaction module 2b in the system 2.
[FIG. 6] FIG. 6 is a diagram illustrating a state that the plate 1 is processed at the position of a fluorometric module 2c in the system 2.
[FIG. 7] FIG. 7 is an enlarged view of an optical system X in the fluorometric module 2c in the system 2.
[FIG. 8] FIG. 8 is a perspective view depicting one example of the layout construction of a first inline header 207 and second inline header 208.
[FIG. 9] FIG. 9 is a plan view of the one example of the layout construction as seen from the top.
[FIG. 10] FIG. 10 is a view showing another example of the layout construction of the inline headers 207, 208.
[FIG. 11] FIG. 11 is a diagram for describing one example of an operation sequence upon feeding (dropping) the medium.
[FIG. 12] FIG. 12 is a graph in which the abscissa and ordinate represent the dropped amount and the drying time, respectively, and the time required until the dropped solution was dried in its entirety is plotted for individual environmental humidities of from 50 to 90% RH.
[FIG. 13] FIG. 13 is an enlarged graph of an extract (around the dropped amount of 100 pL) of the plot for the environmental humidity of 50% RH in FIG. 12.
[FIG. 14] FIG. 14 is a view for describing the procedure of dropwise feeding of a water-supplying solvent to the plate 11.
[FIG. 15] FIG. 15 is a view for describing another dropping procedure for water replenishment (a procedure making use of a camera output image).
[FIG. 16] FIG. 16 is a diagram for describing one example of an operation sequence in the dropping procedure.
[FIG. 17] FIG. 17 is a diagram for describing one example of another operation sequence.
[FIG. 18] FIG. 18 is a diagram for describing one example of a further operation sequence.
[FIG. 19] FIG. 19 is a view useful in describing problems in the conventional art.

### [Best Mode for Carrying out the Invention]

With reference to the accompanying drawings, preferred embodiments of the present invention will hereinafter be described.

It is to be noted that the respective embodiments shown in the accompanying drawings merely exemplify certain representative embodiments of the system and method according to the present invention and the scope of the present invention shall not be narrowly interpreted by these exemplifications.

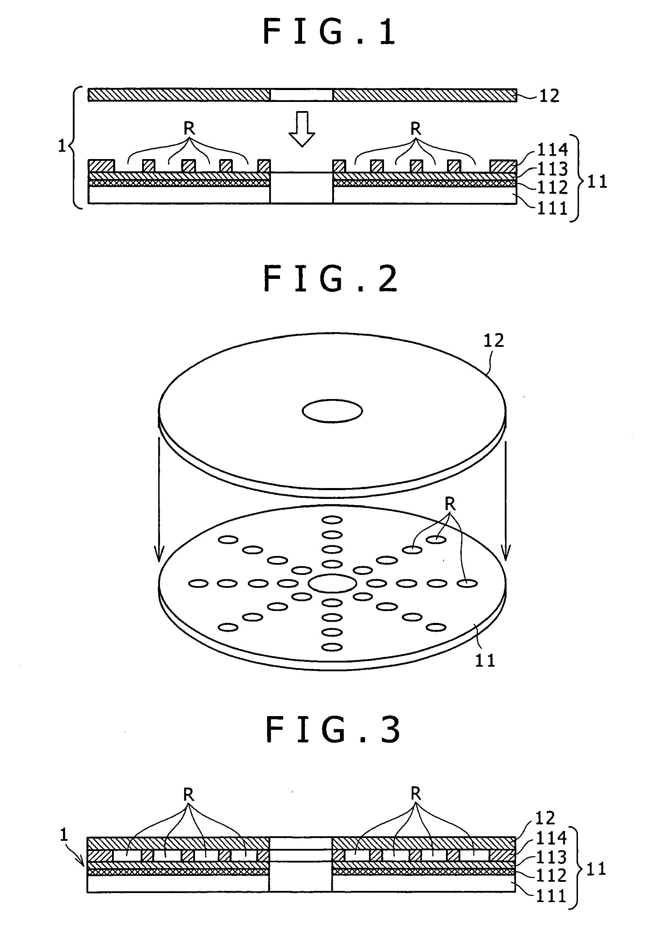

Firstly, FIG. 1 is a cross-sectional view showing the construction of members in a plate with reaction regions to which the bioassay system and method according to the present invention can be applied.

A plate 1 is a plate provided for a bioassay, and is composed of a lower plate 11 and an upper plate (hereinafter called "cover") 12 put on the lower plate by bonding or the like. The lower plate 11 has a layered structure, in which a lower-layer substrate 111, a transparent electrode layer 112, an immobilization layer 113, and a reaction-region defining layer 114 with reaction regions R, for example, in the form of reaction wells, are stacked one over the other.

It is to be noted that the transparent electrode layer 112 in the lower substrate 11 is a layer which can be employed if a certain electrodynamic action is used in the course of a bioassay for the detection of an interaction, and that the transparent electrode layer is not an absolutely essential layer in relation to the present invention.

The lower-layer substrate 111 is formed of a material (for example, synthetic resin or glass) equipped, for example, with such properties as permitting the transmission of laser beams (fluorescence excitation light, position-detecting servolight, etc.) and fluorescence produced in reaction regions R. By providing the lower-layer substrate 111 with light transmission properties, it becomes possible to adopt light irradiation means that irradiates light from the back side of the plate 11.

The transparent electrode layer 112 is formed of a light-transmitting, conductor material, for example, such as indium-tin-oxide (ITO). This transparent electrode layer 112 has been formed with a predetermined film thickness (for example, 200 nm) on the lower-layer substrate 111, for example, by using a sputtering technology.

The immobilization layer 113 is made of a material suited for immobilizing a probe substance, for example, nucleic acid molecules such as probe DNAs (e.g., oligonucleotide chains) at one ends thereof. For example, SiO₂ the surface of which can be modified with a silane has been formed with a predetermined film thickness (for example, 200 nm) by a sputtering technology.

Over a surface layer of the immobilization layer 113, a substance having one or more functional groups (active groups) such as amino groups, thiol groups or carboxyl groups, cysteamine, streptavidin, or the like may be coated. When surface-treated with streptavidin, for example, the immobilization layer is suited for the one-end immobilization of a probe substance such as a biotinylated probe DNA. When surface-treated with thiol (SH) groups, on the other hand, the immobilization layer is suited for the immobilization of a probe substance, which is modified at one end thereof with a thiol group, via a disulfide bonds (-S-S- bonds).

The reaction-region defining layer 114 is a layer, in which the reaction regions R in the form of recesses upwardly open in the lower plate 11 are arranged in a large number. This reaction-region defining layer 114 can be formed, for example, by a photolithography process of a photosensitive polyimide. The reaction regions R may each be formed, for example, in a shape of 100 µm in diameter and 5 µm in depth. In this case, the internal volume of each reaction region R is about 100 pL.

FIG. 2 is a perspective view illustrating how the cover (upper plate) 12 is put on the lower plate 11 of the same diameter, and FIG. 3 is a cross-sectional view depicting the layered construction of the plate 1 obtained by the superimposition of the plate 11 and the cover 12.

The reaction regions R are arrayed in a plural number such that, when the lower plate 11 is seen as a whole from the top as shown in FIG. 2, they present a radial pattern from a center of the plate. Further, the radial arrays of the reaction regions R are in such a form that these reaction regions are arranged at predetermined radial intervals.

In one example of the lower plate 11, the one example having been actually prepared by the present inventors, reaction regions R as many as 50 in total were formed at 0.2 mm intervals between 25 mm and 35 mm in radius from the center of the plate to present a radial array, and such radial arrays were arranged as many as 785 arrays at 0.2 mm pitches in the radial direction. In that example, the reaction regions R as many as 39,250 in total (50 reaction regions x 785 arrays) were, therefore, formed on the plate.

Although not specifically shown in any drawing, address pits, bar codes, or the like which function as position information (address information) on the plate 1 are formed on the plate. For example, an address pit indicative of a reference position in the direction of rotation can be formed on the reaction-region defining layer 114 by a similar process as the reaction regions R. By tracking this address pit with predetermined servolight, positional information on the plate can be obtained. Relying upon this positional information on the plate, each target reaction region R can be specified exactly.

The cover 12 primarily functions as a member for preventing drying of a medium stored or held in the reaction regions R. As illustrated in FIG. 3, for example, when the cover 12 is put on, it comes into close contact such that the reaction regions 3 are enclosed and are maintained out of communication with the air. For example, the cover 12 can be made of n-type Si having electrical conductivity.

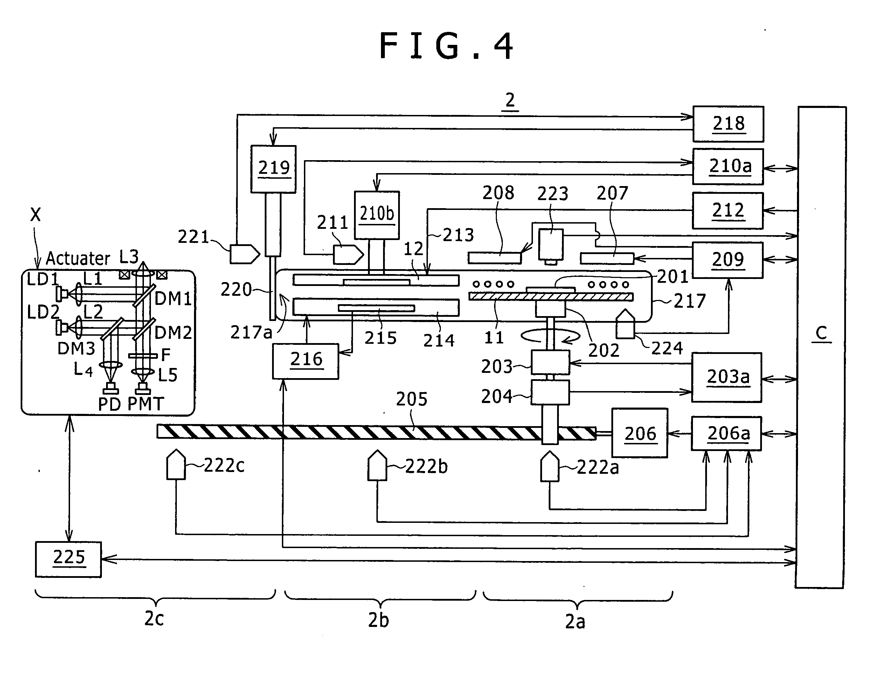

Based on FIG. 4 through FIG. 8, a description will next be made about a "bioassay system" according to the present invention.

Roughly speaking, the "bioassay system" designated at numeral 2 in FIG. 4, etc. is constructed of a feed module 2a for feeding a medium into the reaction regions R, a reaction module 2b for allowing an immobilization reaction or interaction to proceed, and a fluorometric module 2c for measuring excited fluorescence in the reaction regions R. It is to be noted that in the system 2, drivers (to be described subsequently herein) for controlling respective operations, the application of an electric field, and the like are controlled by a computer C.

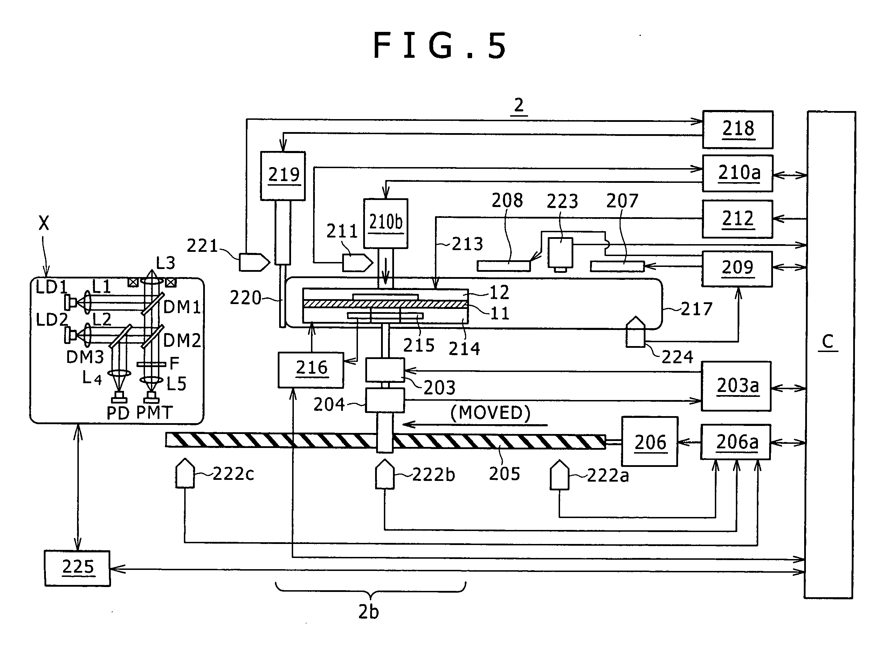

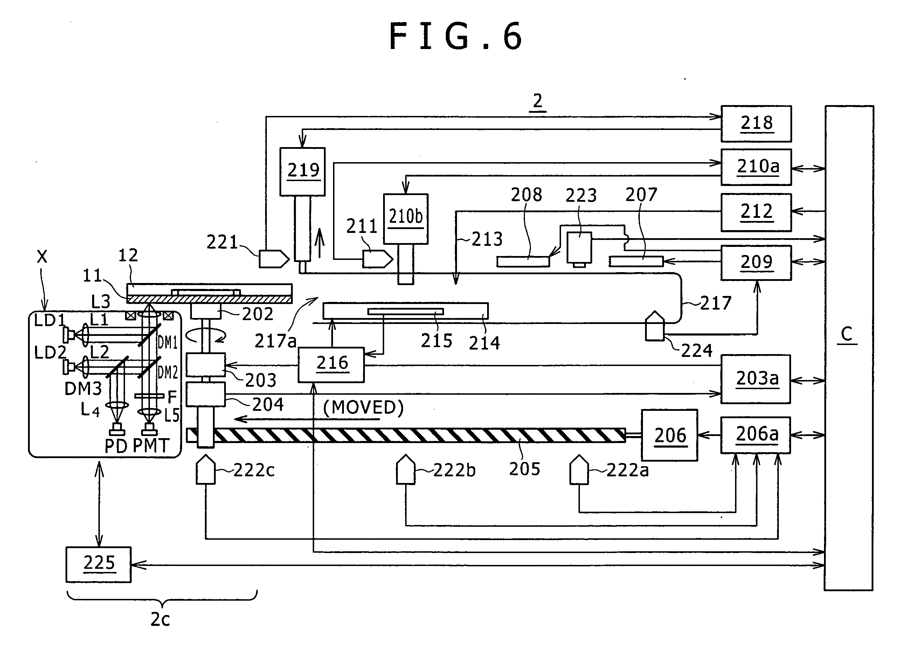

FIG. 4 illustrates a state that the lower plate 11 is being subjected to treatment at the position of the feed module 2a, FIG. 5 shows a state that the plate 1 is being subjected to treatment at the position of the reaction module 2b, and FIG. 6 depicts a state that the plate 1 is subjected to treatment at the position of the fluorometric module 2c.

As is appreciated from the foregoing, the lower plate 11 is constructed such that it slidingly moves among the respective modules 2a, 2b, 2c and stops at predetermined positions in the respective modules 2a, 2b, 2c to perform the intended treatment or reaction.

The lower plate 11 of the above-described construction is fixedly mounted on a turntable 202 via a chucking mechanism 201 shown in FIG. 4, etc. The turntable 202 is connected to a spindle motor 203 and a rotary encoder 204. The spindle motor 203 is in meshing engagement with a feed screw 205 so that by a spindle stepper motor 206 controlled by a driver 206a, the plate 1 can be conveyed into the respective modules of the feed module 2a, reaction module 2b, and fluorometric module 2c.

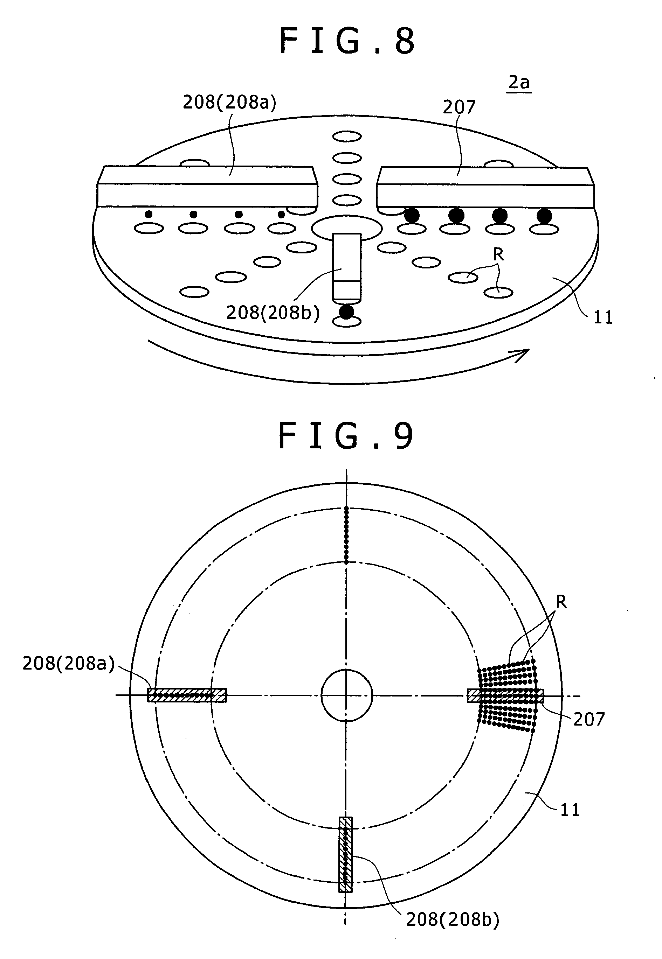

As illustrated in FIG. 8, the feed module 2a is provided in an area over the lower substrate 11 with a first inline header 207 in which fifty inkjet nozzles as many as the reaction regions R in each radial ray are arranged via a detachable mechanism. This first inline header 207 functions as medium feeding means for the reaction regions R.

This first inline header 207 is constructed such that by replacing feeding inkjet nozzles filled with a probe-substance-containing medium (not shown) with feeding inkjet nozzles filled with a target-substance-containing medium (not show) or vice versa as desired, the medium with the desired substance contained therein can be fed into the reaction regions R.

In addition to the above-described first inline header 207 for feeding the medium with the probe substance or the like contained therein, the feed module 2a is also provided with as many second inline header(s) 208 as needed. The second inline header(s) 208 is (are) arranged exclusively for feeding water into the reaction regions R on the lower plate 11, and function(s) as water replenishing means for the reaction region R.

The second inline header(s) 208 is (are each) provided with inkjet nozzles as many as 50 in total, which is the same number as the group of reaction regions R in each radial array on the lower plate 11. It is to be noted that the inline headers 207, 208 are both connected to and controlled by an inkjet driver 209.

The reaction module 2b is equipped with a mechanism that superimposes and mounts the conductive cover 12, which plays a role such as the prevention of drying, on the lower plate 11 on which the reaction regions R are arranged. This mounting mechanism is constructed primarily of an actuator driver 210a for controlling the attachment and detachment of the cover 12, an actuator 210b controlled by the driver 210a, a position sensor 211 for detecting the position of the cover 12, etc.

This reaction module 2b is equipped with a contact electrode 213 for applying an electric field such as a high-frequency a.c. field to the electrically-conductive cover 12 via a power supply 212 such as a high-frequency power supply, a heater 214 for heating the plate 1, etc. Further, numeral 215 is a temperature sensor arranged on the heater 214, and numeral 216 is a heater driver for controlling the temperature of the heater 214 in response to a detection signal from the temperature sensor 215.

The lower plate 11 and cover 12 and the heater 214 can all be held within a constant-humidity chamber 217. This constant-humidity chamber 217 has an opening 217a at a part thereof. This opening 217a is opened or closed by moving a shutter 220 upward or downward via an actuator 219 controlled by a driver 218. Designated at numeral 221 is a position sensor for detecting the position of the shutter 220. It is to be noted that the constant-humidity chamber 217 is kept closed except when the plate (or the lower plate 11) passes upon its conveyance (see, for example, FIG. 4).

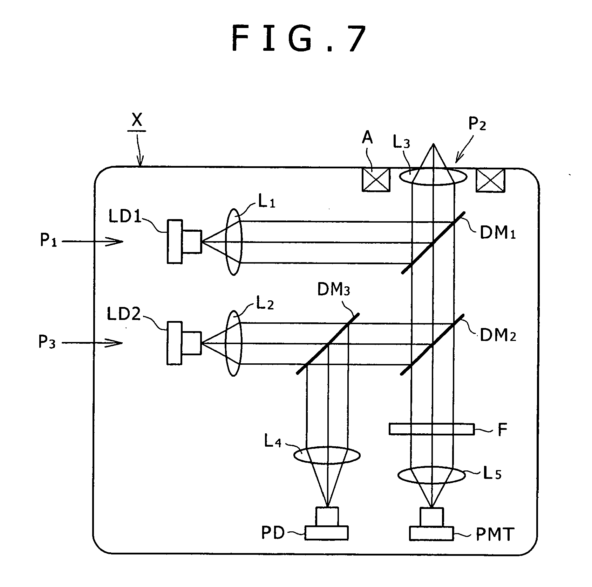

The fluorometric module 2c is composed primarily of an optical system X controlled by a measurement control system 225. As shown in FIG. 7 in which the optical system X of FIG. 4 to FIG. 6 is illustrated on an enlarged scale, the optical system X is equipped with a fluorescence-excitation optical system P1 (fluorescence excitation laser LD1, collimator lens L1, objective lens L3, dichroic mirror DM1) for effecting fluorescence excitation of a phosphor which exists within the reaction regions R on the lower substrate 11 (for example, a fluorescent dye or fluorescent intercalator labeled on the target substance).

The optical system X is also equipped with a fluorometric optical system P2 for measuring excited fluorescence (objective lens L3, wavelength selective mirror F, objective lens L5, fluorometric detector PMT, dichroic mirrors DM1, DM2), and further with an AF detection optical system P3 for detecting an autofocus AF control signal for the objective lens L5 (AF detection laser LD2, collimator lens L2, beam splitter M3, astigmatic lens L4, AF detector PD).

The fluorescence excitation laser, AF detection laser, and fluorescence have different wavelengths, respectively, and are combined/split through the dichroic mirrors DM1, DM2.

In the feed module 2a and reaction module 2b, the above-described constant-humidity chamber 217 is arranged (see FIG. 4, etc.), and by an unillustrated constant-humidity controller, the plate-surrounding environment is maintained at a constant humidity, for example, 50% RH. As a result, the loss (evaporation) of water from the probe-substance-containing medium or target-substance-containing medium after its feeding can be controlled minimum.

A description will now be made about an assay on the immobilization of a probe substance, which is performed by using the bioassay system 2 of the above-described construction. The description will hereinafter be made by taking a probe DNA as a representative example of the probe substance. It should, however, be borne in mind that the following description should not be interpreted as limiting the probe substance to the probe DNA.

The probe DNA is a single-stranded DNA (nucleotide chain) synthesized to have a base sequence complementary to a base sequence the inclusion or non-inclusion of which is desired to be determined in a target DNA (single strand) as a target substance in a below-described bioassay process.

The feeding of the probe DNA into the reaction regions R arranged on the lower plate 11 is conducted, at the position of the feed module 2a, by feeding (dropping) predetermined amounts of a medium with the probe DNA contained therein into the reaction regions R via the inkjet nozzles arranged on the first inline header 207.

The inkjet nozzles which function as feeding nozzles, including both of those for the probe-containing medium and those for a solvent for the replenishment of water, are arranged at the same pitch and as many as the reaction regions R in each radial array formed on the lower plate 11 so that the different media can be dropped from the nozzles, respectively.

Firstly, the lower plate 11 (in an uncovered state) is mounted on the turntable 202 at the position of the fluorometric module 2c. After the plate 11 is fixed by the chucking mechanism 201, the plate 11 is conveyed by the spindle stepper motor 206 to the position of the feed module 2a as detected by a spindle position sensor 222a. The state of the plate 11 after the conveyance is illustrated in FIG. 4.

The lower plate 11 is then rotated by the spindle motor 203 controlled by a driver 203a, and from an output of a disk reference position sensor 223 for detecting the reference position of the lower plate 11 in the direction of rotation and an output of the rotary encoder 204, a signal indicative of the corresponding reaction region R (reaction-region position signal) is produced. By synchronizing the reaction-region position signal and a delivery position signal for the corresponding feeding nozzle, the medium with the intended probe DNA contained therein is fed into the desired reaction region R on the lower plate 11.

In FIG. 8 and FIG. 9, one example of the layout construction of the inline headers 207, 208 is illustrated. FIG. 8 is a perspective view, while FIG. 9 is a plan view as seen from the top.

In the example depicted in FIG. 8 and FIG. 9, inkjet nozzles of 100 pL delivery rate and 5 kHz delivery frequency are adopted as the inkjet nozzles filled with the probe-DNA-containing medium. For example, the inkjet nozzles as many as 50, which is the same number as that of the reaction regions R in each radial array, are arranged on the first inline header 207 via the detachable mechanism.

On the other hand, inkjet nozzles of 2 pL delivery rate and 20 kHz delivery frequency, for example, are adopted as the water-replenishing inkjet nozzles. For example, the inkjet nozzles as many as 50, which is the same number as that of the reaction regions R in each radial array, are arranged on each of the second inline header 208 via the detachable mechanism.

In this example, the two inline headers 208 are arranged (see FIG. 8 and FIG. 9). Their roles are divided, that is, one (208a) being for the adjustment of the concentration of the substance in the medium, and the other (208b) for the prevention of the precipitation of the substance.

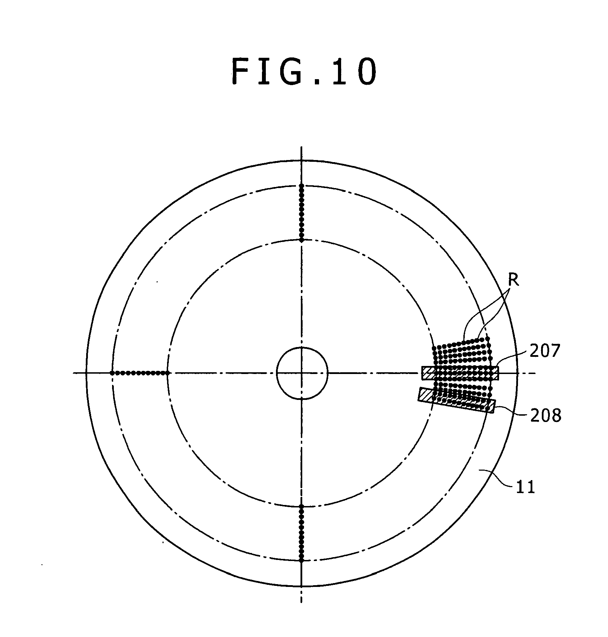

It is to be noted that as in a modification illustrated in FIG. 10, both of the substance-concentration-adjusting function and the substance-precipitation-preventing function may be on a second inline header 208 or only one of these roles may be assigned to the second inline header 208.

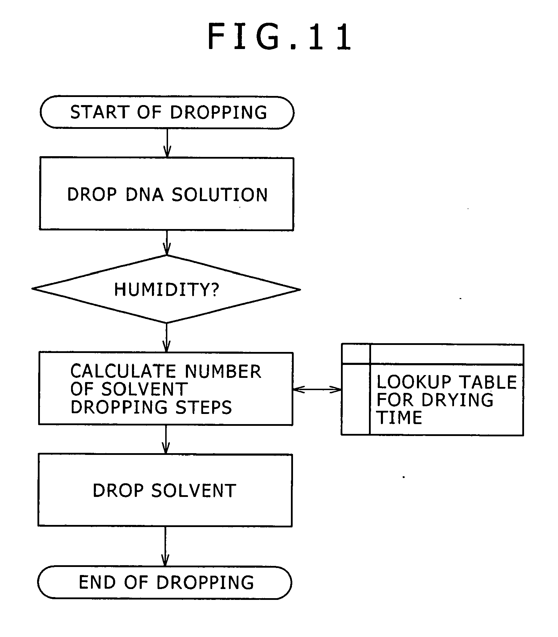

Based on FIG. 11, a description will next be made about an operation sequence upon feeding (dropping) the medium.

Firstly, the lower plate 11 is rotated, and the medium with the probe DNA contained therein is fed dropwise into reaction regions R on the plate 11. The plate 11 is then rotated. At a timing that the reaction regions R have come to the position of the water-replenishing second inline header 208, the number of solvent dropping steps is calculated with reference to a "lookup table for drying time" stored in the computer C, and the water-replenishing solvent is fed dropwise as much as the required dropping steps.

This "lookup table for drying time" was prepared beforehand from an experiment which had been conducted in advance under respective humidity conditions. Corresponding to the position numbers of the respective reaction regions R, the numbers of water-replenishing solvent dropping steps are recorded in the "lookup table for drying time".

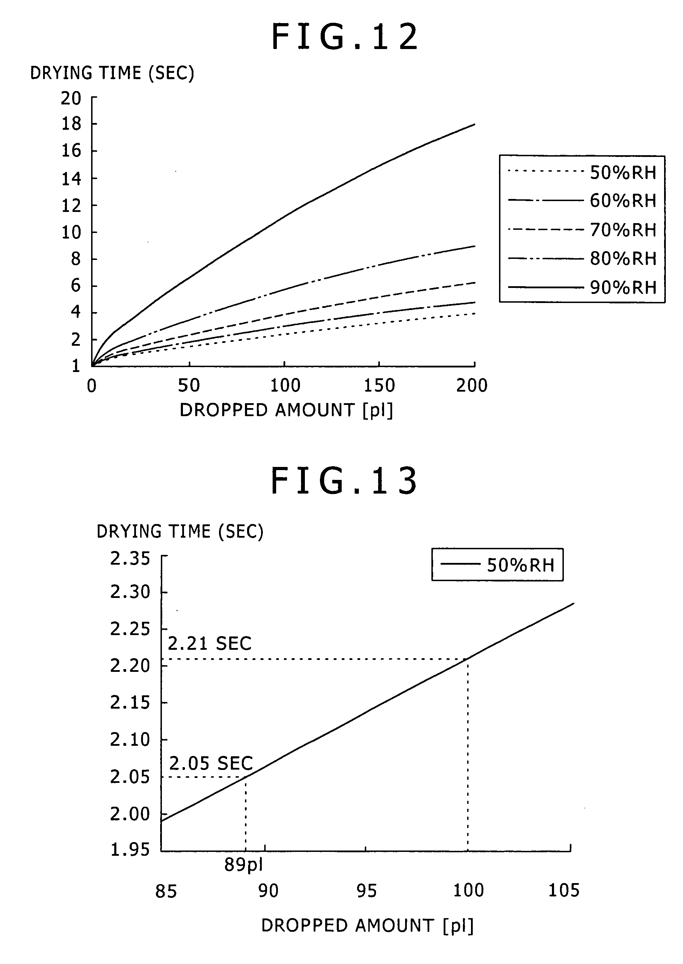

FIG. 12 and FIG. 13 diagrammatically show the data obtained by the experiment. FIG. 12 is a graph in which the abscissa and ordinate represent the dropped amount and the drying time, respectively, and the time required until the dropped solution was dried in its entirety is plotted for individual environmental humidities of from 50 to 90% RH. FIG. 13 is an enlarged graph of an extract (around the dropped amount of 100 pL) of the plot for the environmental humidity of 50% RH in FIG. 12.

According to that experiment, it is appreciated that in the case of 50% RH humidity, for example, about 11 pL of water had been lost through drying from the probe-DNA-containing medium held in each of the reaction regions R, into which the medium was dropped first (for example, in a reaction region array 1), upon elapsed time of 0.16 second from the dropping into the last reaction regions R (for example, in a reaction region array 785).

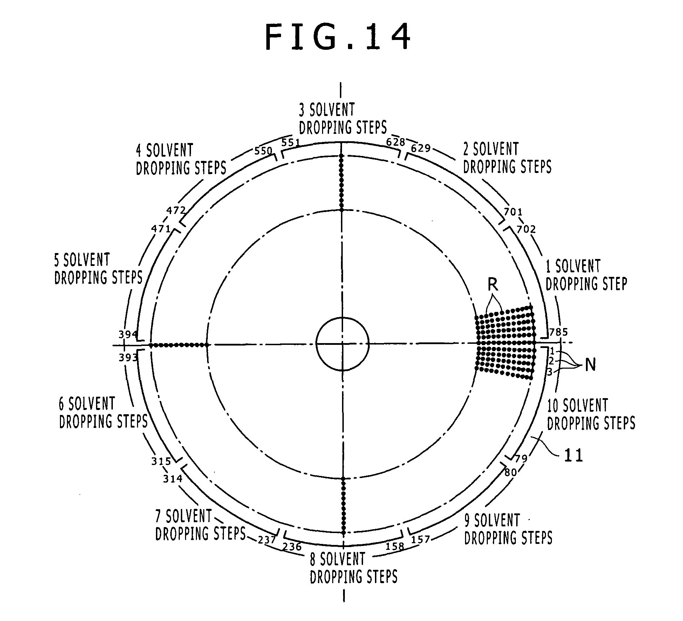

As illustrated in FIG. 14, it is hence possible to control minimum a change through drying in the concentration of the probe DNA in each reaction region R by calculating and setting a number of solvent dropping steps for each reaction region array number.

More specifically, array numbers (see letter Ns) are allotted to the reaction regions R, which are arrayed on the plate 11 to present radial arrays, radial array by radial array, one after another in a circumferential direction. To each of the reaction regions R existing as a group in an area specified by an array number N, the water-replenishing solvent is fed as much as the required dropping steps (see FIG. 14). It is to be noted that in FIG. 14, the later the feeding order, the more the dropping steps.

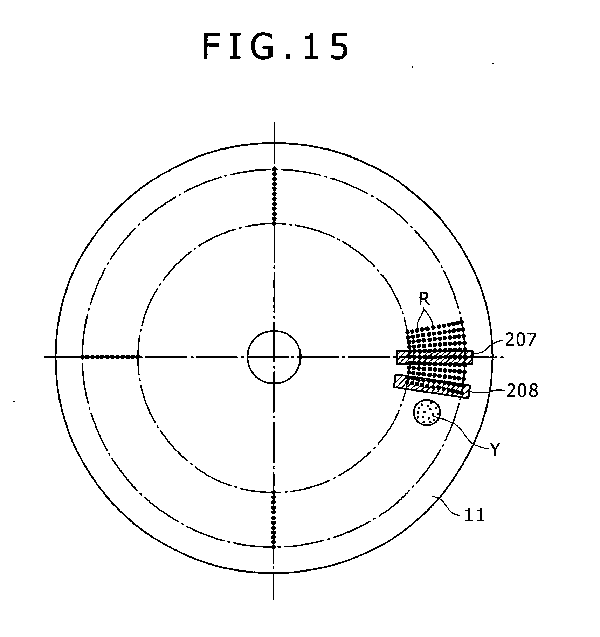

With reference to FIG. 15, a description will next be made about another embodiment of the water replenishment.

In this embodiment, the inkjet nozzles in which the probe-DNA-containing medium is filled are assumed to be of 100 pL delivery rate and 5 kHz delivery frequency. The inkjet nozzles as many as the number of reaction regions R in each radial ray (for example, 50) are mounted on the first inline header 207 via a detachable mechanism.

In this embodiment, the solvent-feeding inkjet nozzles which serve to replenish water are assumed to be of 2 pL delivery rate and 20 kHz delivery frequency. These inkjet nozzles are mounted in the same number (50) as the number of the reaction regions R in one of the radial arrays of reaction regions R on the second inline header 208 via a detachable mechanism.

In this embodiment, an optomicroscopic CCD camera 223 useful for the automated measurement of the amount of a medium in each reaction regions R is arranged above the plate 11 (see FIG. 4 to FIG. 6). It is to be noted that a range designated by numeral Y in FIG. 15 indicates a focal area of the optomicroscopic CCD camera 223.

The volume of the medium in each reaction region R can be calculated from dimensions of a meniscus profile of the medium by extracting the profile of the solution from a camera output image.

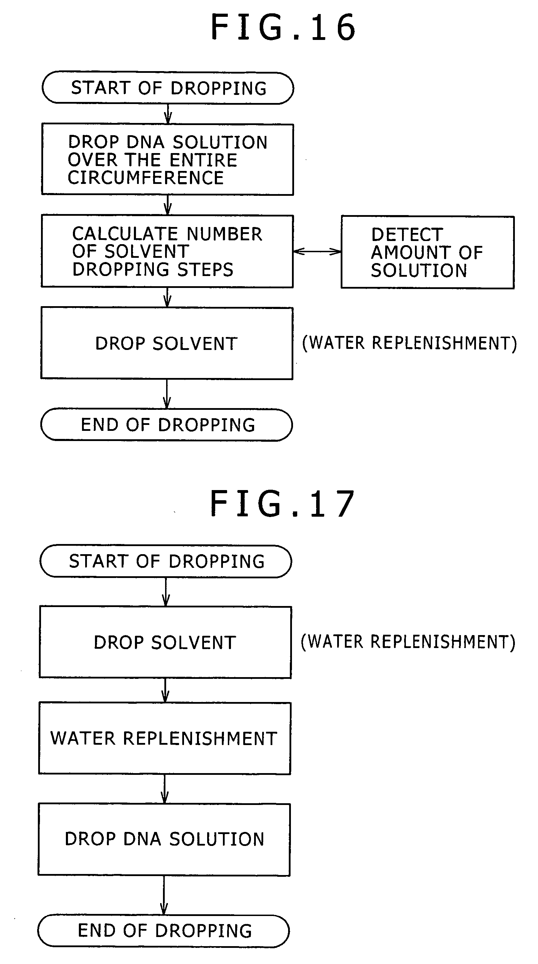

An example of an operation sequence in this embodiment will be described based on FIG. 16.

The lower plate 11 is rotated, and the probe-DNA-containing medium is fed (dropped) into all the reaction regions R on the plate 11. In the next rotation, the amount of the medium in each reaction region R is detected by using the optomicroscopic CCD camera 223, and then, a calculation is performed to determine the amount of a decrease in the solution. Based on this calculation, a water-replenishing solvent is dropped by performing as many dropping steps as needed at a timing that the specific reaction region R has moved to the position of the water-replenishing second inline header 208.

Owing to this construction, a change through drying in the concentration of the probe DNA in each reaction region R can be controlled minimum without detecting beforehand the humidity in the constant-humidity chamber 217 (see FIG. 4, etc.).

In a further embodiment, inkjet nozzles of 2 pL delivery rate and 20 kHz delivery frequency may be adopted as inkjet nozzles for feeding the probe-DNA-containing medium. These inkjet nozzles are mounted as many as the number of the reaction regions R in one of the radial arrays of reaction regions R (for example, 50 inkjet nozzles) on the first inline header 207 via a detachable mechanism.

In this embodiment, inkjet nozzles of 100 pL delivery rate and 5 kHz delivery frequency are adopted as water-replenishing (solvent-dropping) inkjet nozzles. These inkjet nozzles are mounted as many as the number of the reaction regions R in one of the radial arrays of reaction regions R (for example, 50 inkjet nozzles) on the first inline header 207 via a detachable mechanism.

A feed operation sequence in this case will be described based on FIG. 17.

Firstly, the plate 11 is rotated and the water-replenishing solvent is fed dropwise beforehand via the second inline header 208. Next, the plate 11 is rotated, and the probe-DNA-containing medium is fed dropwise into the reaction regions R via the first inline header 207 on which nozzles for feeding dropwise the probe-DNA-containing medium are arrayed. The concentration of the probe-DNA-containing medium should be adjusted beforehand such that a predetermined concentration will be reached when mixed with 100 pL of the solvent in each reaction region R.

When an assay is performed as described above, no complete mixing takes place in the short time from the dropwise feeding of the solution until the mounting of the drying-preventing cover (upper plate 12), thereby making it possible to effectively prevent the probe DNA from precipitating or sticking in the reaction regions R.

By dropping the probe-DNA-containing medium subsequent to the dropping of the solvent into the reaction regions R, irregularities caused by the precipitation or sticking of the probe substance in the reaction regions R can be reduced, and therefore, a high-accuracy bioassay can be realized.

In a still further embodiment, inkjet nozzles of 2 pL delivery rate and 20 kHz delivery frequency are adopted as inkjet nozzles for the first inline header 207. In addition, inkjet nozzles of 100 pL delivery rate and 5 kHz delivery frequency are adopted as inkjet nozzles for the (water-replenishing) second inline header 208. By using the photomicrographic CCD camera 223 for measuring the amount of the medium in each reaction region R, the amount of the medium in the reaction region R is calculated from dimensions of a meniscus profile of the medium by extracting the profile of the solution from a camera output image.

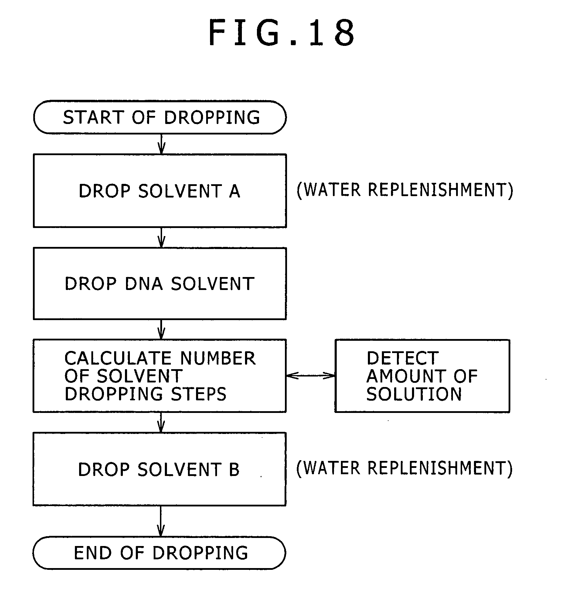

A feeding operation sequence in this case will be described based on FIG. 18.

Firstly, the plate 11 is rotated, and a solvent A is fed dropwise into reaction regions R. The plate 11 is then rotated. At a timing that the reaction regions R have come to the position of the first inline header 207, a probe-DNA-containing medium is fed dropwise into the reaction regions R. The above-described feeding operation is performed over the entire circumference of the plate, and in the next rotation, the amount of the medium held in each reaction region R is detected by using the optomicroscopic CCD camera 223 and the amount of its decrease is calculated. Based on the calculation results, a solvent B is added dropwise by performing as many dropping steps as needed when the specific reaction region R has come to the position of the second inline header 208 that serves to perform the dropwise feeding of the solvent B.

According to this method, a change through drying in the concentration of the probe DNA in each reaction region R can be controlled minimum without detecting beforehand the humidity in the constant-humidity chamber 217 (see FIG. 4, etc.). It is also possible to effectively prevent the probe DNA from precipitating or sticking in the reaction region R.

After the probe-DNA-containing medium is fed dropwise onto the plate 11 by the method indicated in any one of the above-described embodiments, the plate 11 is conveyed by the spindle stepper motor 206 to the position of the reaction module 2b as detected by a spindle position sensor 222b (the state of FIG. 5). The cover (upper plate 12) is then mounted on the plate 11 by using the cover mounting/dismounting actuator 210b and the cover position sensor 211 (see FIG. 5 again).

Subsequently, the plate 11 is placed standstill for a certain time within the constant-humidity chamber 217 to complete the immobilization work of the probe DNA on the surfaces (surfaces treated for immobilization) of the reaction regions R.

The plate 11 on which the immobilization work of the probe DNA has been completed is conveyed to the position of the fluorometric module 2c as detected by a spindle position sensor 222c. The plate 11 is dismounted from the turntable 202. By feeding a desired washing solution into the reaction regions R and discharging it from the reaction regions R, washing treatment is applied to eliminate any probe DNA which may still remain in a non-immobilized state in the reaction regions R. At a predetermined place, the plate 11 is then stored ready for a bioassay process.

A description will hereinafter be made about the bioassay process after the immobilization. This bioassay process means a series of steps of dropwise feeding of a target substance progress of an interaction (for example, hybridization fluorometric process.

Firstly, the target substance (which is now assumed to be a "target DNA") is a single-stranded DNA (nucleotide chain) which is desired to be investigated as to whether or not a base sequence complementary to the probe DNA is contained, and is extracted and isolated from an organism.

The lower plate 11 with the probe DNA immobilized thereon is mounted on the turntable 202 at the position of the fluorometric module 2c, and is fixed by the chucking mechanism 201. The lower plate 11 is then conveyed by the spindle stepper motor 206 to the position of the feed module 2a as detected by the spindle position sensor 222a (see the state of FIG. 4).

The inkjet nozzles are filled beforehand, for example, with a medium (for example, a solution) which contains the target DNA and an intercalator (which will be described subsequently herein). The lower plate 11 is rotated by the spindle motor 203, and from an output of the disk reference position sensor 224 that serves to detect the reference position in the direction of rotation on the lower plate 11 and an output of the rotary encoder 204, a signal indicative of the corresponding reaction region is produced. By synchronizing the position signal indicative of the reaction region R and a delivery signal for the corresponding dropping nozzle, the medium is fed dropwise into the desired reaction region R formed on the lower plate 11. The feeding operation at this time can be performed by a similar procedure as in the above-described FIG. 16. In this case, the "DNA solution" shown in FIG. 16 means the target-DNA-containing solution.

In the target-DNA-feeding work, a water-replenishing solvent is also fed dropwise into the reaction regions R. Described specifically, at a timing that the reaction regions R in desired one of the radial arrays have come to the position of the second inline header 208, the solvent is dropped into each reaction region R by performing as many dropping steps as needed while referring to the lookup table for drying time which has been obtained beforehand by the above-described experiment. In this manner, a change through drying in the concentration of the target DNA in each reaction region R can be controlled minimum.

In the target-DNA-feeding work, the use of the photomicrographic CCD camera 223, which is useful in measuring the amount of the medium in each reaction region R, also makes it possible to calculate the amount of the medium in the reaction region R from dimensions of a meniscus profile of the medium by extracting the profile of the solution from a camera output image.

An operation sequence in the above case is similar to that of FIG. 16 described above. Described specifically, the plate 11 is rotated, and a target-DNA-containing medium (which corresponds to the DNA solution in FIG. 16) is fed dropwise over the entire circumference. In the next rotation, the amount of the medium in each reaction region R is detected, and a calculation is performed to determine the amount of the medium decreased through drying. When the selected reaction region R has come to the position of the second line header 208, the solvent is fed dropwise by performing as many dropping steps as needed. In this manner, a change through drying in the concentration of the target DNA in each reaction region R can be controlled minimum without detecting beforehand the humidity in the constant-humidity chamber 217.

In the feeding of the target DNA, it is also possible to adopt such an operation sequence as illustrated in FIG. 17. Described specifically, the plate 11 is rotated and the solvent is dropped beforehand. Subsequently, at a timing that the reaction regions R in desired one of the radial arrays have come to the position of the first inline header 207, the target-DNA-containing medium is fed dropwise. The concentration of the target-DNA-containing medium should be adjusted beforehand such that a predetermined concentration is reached when the target-DNA-containing medium is mixed with 100 pL of the solvent in the reaction region R. By conducting such a method, it is possible to effective prevent the target DNA from precipitating or sticking in the reaction region R.

In the target-DNA-feeding work, the amount of the medium in the reaction region R can also be calculated from dimensions of a meniscus profile of the medium by extracting the profile of the solution from a camera output image acquired by the photomicrographic CCD camera 223 which is useful in measuring the amount of the medium in the reaction region R.

An operation sequence in this case is similar to that of FIG. 18 described above. Described specifically, the plate 11 is rotated to firstly feed a solvent A dropwise into reaction regions R. The plate 11 is then rotated. At a timing that the reaction regions R in desired one of the radial arrays have come to the position of the first inline header 207, a target-DNA-containing medium is fed dropwise. The above-described feeding operation is performed over the entire circumference of the plate, and in the next rotation, the amount of the medium held in each reaction region R is detected by using the optomicroscopic CCD camera 223 and the amount of its decrease is calculated. Based on the calculation results, a solvent B is added dropwise by performing as many dropping steps as needed when the specific reaction region R has come to the position of the second inline header 208 that serves to perform the dropwise feeding of the solvent B.

According to this method, a change through drying in the concentration of the probe DNA in each reaction region R can be controlled minimum without detecting beforehand the humidity in the constant-humidity chamber 217 (see FIG. 4, etc.). It is also possible to effectively prevent the probe DNA from precipitating or sticking in the reaction region R.

After the target-DNA-containing medium is fed dropwise as described above, the plate 11 is conveyed by the spindle stepper motor 206 to the reaction module 2b as detected by the spindle position sensor 222b. The drying-preventing cover (upper plate 12) is then mounted on the plate 11 by using the cover mounting/dismounting actuator 210b and the cover position sensor 211 (see the state of FIG. 5).

The plate 11 is now left over for a certain time within the reaction module 2b. If a base sequence complementary to the (immobilized) probe DNA is contained in the target DNA, they undergo hybridization to form a double-stranded DNA.

This hybridization process is performed in such a state that concurrently with the mounting of the cover 12, the plate 11 is brought into contact under pressure with the heater 214 and is heated, for example, at 55. Further, a high-frequency a.c. field of 1 MV/m and 1 MHz, for example, may be applied to the reaction regions R by using the transparent electrode layer 112 (see FIG. 1) in the lower plate 11 and the electrically-conductive cover (upper plate) 12 as opposing electrodes and connecting them to the high-frequency power supply 212.

The application of the electric field to the reaction regions R is intended to stretch or migrate nucleic acid molecules under electrodynamic effects such as dielectrophoresis. The application of the electric field makes it possible to avoid a steric hindrance upon hybridization or to increase the association probability between the prove DNA and the target DNA. As a result, the hybridization can be performed promptly.

After completion of the hybridization between the probe DNA immobilized in the reaction regions R and the target DNA, the lower plate 11 with the cover (upper plate) 12 mounted thereon is conveyed to the position of the fluorometric module 2c as detected by the spindle position sensor 222c (see the state of FIG. 6).

It is to be noted that the intercalator fed into the reaction regions R is a phosphor having a property that it modifies into a fluorescence-emitting structure when bonded to a double-stranded DNA. Accordingly, this intercalator emits fluorescence upon its bonding to a double-stranded DNA formed when the target DNA has a base sequence complementary to the probe DNA.

It is, therefore, possible to determine the inclusion or non-inclusion of a specific base sequence in the target DNA by measuring the intensity of fluorescence from the intercalator. Although no particular limitation is imposed on the intercalator, commercially-available SYBERGreen I or the like can be adopted, for example.

Fluorometry can be performed by a similar operation as in conventional optical disk systems. Described specifically, the lower plate 11 is rotated by the spindle motor 203, and the position of the objective lens L3 relative to the surface of the plate is controlled by the AF detection optical system P3 and the actuator (see FIG. 7, in particular).

The intercalator in each reaction region R on the plate is then excited by the fluorescence-excitation optical system P1, and fluorescence from the intercalator is measured by the fluorometric optical system. At this time, the fluorescence excitation laser LD1 uses a semiconductor laser of 450 nm wavelength, which is converted into a parallel beam through the collimator lens L1, and after being deflected by the following dichroic mirror DM1, is focused through the objective lens L3 onto the immobilization layer 113 in the reaction region R to excite the intercalator bonded to the double-stranded DNA formed on the immobilization layer 113 (see FIG. 7).

This intercalator produces fluorescence around 520 nm wavelength. The fluorescence passes through the objective lens L3 and dichroic mirrors DM1, DM2, and subsequent to elimination of stray light through the wavelength selective mirror F, is focused through the objective lens L5 onto a light-receiving portion of the fluorometric detector PMT. As a consequence, the intensity of the fluorescence is measured.

As the fluorescence is weak at this time, it is desired to adopt a photomultiplier as the fluorometric detector PMT. Further, the AF detection optical system P3 and a lens actuator A (see FIG. 7) can use the constructions and control methods, which are used for optical disks, as they are.

This embodiment adopts a construction that the AF detection laser LD2 uses a semiconductor laser of 780 nm wavelength, the semiconductor laser is converted into a parallel beam through the collimator lens L2, and the parallel beam is allowed to travel via the beam splitter M3, deflected by the dichroic mirror DM2, allowed to travel via the dichroic mirror DM1, focused through the objective lens L3 onto the surface of the plate, and then reflected by the surface of the plate (see FIG. 7).

The reflected laser beam travels via the objective lens L3 and dichroic mirrors DM1, DM2, and reaches the beam splitter M3. The laser beam is then deflected into a focus error detection optical system composed of the astigmatic lens L4 and AF detector PD and making use of an astigmatic method.

The fluorometric control system firstly uses the AF detection optical system P3 to detect the reference position mark indicative of the reference position in the direction of rotation on the outermost circumference of the plate, and from an output of the rotary encoder 204 (see FIG. 4, etc.), stores a reference position signal, and calculates the position of each reaction region R.

The plate 1 is then rotated, and the position of the objective lens L3 is subjected to autofocus control by the actuator A to permits stable fluorometry at the position of each reaction region R.

By analyzing the intensity of fluorescence obtained by the measurement, the base sequence contained in the target DNA, that is, its genetic information can be analyzed. In this manner, a series of bioassays can be realized. In the foregoing, the descriptions were made by referring to nozzles of the inkjet system as the medium-feeding nozzles. However, any nozzles can be adopted insofar as they are delivery means capable of dropping or injecting a medium in accurate volumes.

### [Industrial Applicability]

The present invention can be used as a technology for effectively preventing a change in the concentration of a contained substance or precipitation or sticking of a substance in a medium, which would otherwise take place as a result of drying of a medium stored or held in a reaction region that provides a field for an interaction between substances. The present invention can be used as a bioassay technology applicable to sensor chips such as DNA chips and protein chips.

## Claims

1. A bioassay system comprising at least:
medium feeding means for feeding, to reaction regions that provide fields for an interaction between substances, a medium that contains one of said substances to be involved in said interaction, and
water replenishing means for automatically replenishing water into said reaction regions as needed.

2. The bioassay system according to claim 1, further comprising volume detecting means capable of automatically detecting a volume of said medium held in each of said reaction regions such that based on information on a volume change available from said volume detecting means, water is replenished in an amount corresponding to that lost through drying to said each reaction region via said water replenishing means.

3. The bioassay system according to claim 1, wherein said interaction is hybridization between nucleic acid molecules.

4. The bioassay system according to claim 2, wherein said volume detecting means extracts a profile of said medium held in said each reaction region from a camera output image, and calculates said volume of said medium from dimensions of a meniscus profile of said medium.

5. A bioassay method for performing a step of feeding, to reaction regions that provide fields for an interaction between substances, a medium, containing one of said substances to be involved in said interaction, and water via different routes by one of the following procedures (1) and (2):
(1) feeding said medium that contains said one of said substances to be involved in said interaction, and then replenishing said water, and
(2) replenishing said water, and then feeding said medium that contains said one of said two substances to be involved in said interaction.

6. The bioassay method according to claim 5, wherein said one of said substance to be involved in said interaction is a probe substance to be immobilized in said reaction regions or a target substance to be interacted with said probe substance.
